# EUROPÄISCHE PATENTSCHRIFT

(11) **EP 1 448 522 B1**
(45) Veröffentlichungstag und Bekanntmachung des Hinweises auf die Patenterteilung: **28.01.2009**
(21) Anmeldenummer: 02803357.9
(22) Anmeldetag: 07.11.2002
(51) Int. Cl.: C07D 207/44

(54) **GABUSECTIN-DERIVATE, VERFAHREN ZUR DEREN HERSTELLUNG UND DEREN VERWENDUNG**
GABUSECTIN DERIVATIVES, METHOD FOR THE PRODUCTION THEREOF AND USE OF THE SAME
DERIVES DE GABUSECTINE, LEUR PROCEDE DE PREPARATION ET LEUR UTILISATION

(30) Priorität: 21.11.2001 DE 10156906
(43) Veröffentlichungstag der Anmeldung: 25.08.2004
(73) Patentinhaber: Sanofi-Aventis Deutschland GmbH, 65929 Frankfurt am Main (DE)
(72) Erfinder: SCHIELL, Matthias, 65611 Brechen (DE); KNAUF, Martin, CH-6037 Root (CH); TOTI, Luigi, 65239 Hochheim (DE); MARKUS-ERB, Astrid, 65835 Liederbach (DE)
(86) Internationale Anmeldenummer: PCT/EP2002/012420
(87) Internationale Veröffentlichungsnummer: WO 2003/043984

(56) Entgegenhaltungen:
- WO-A-01/74772
- WO-A-02/46152
- ROYLES B J L: "Naturally Occuring Tetramic Acids: Structure, Isolation, and Synthesis" CHEMICAL REVIEWS, AMERICAN CHEMICAL SOCIETY. EASTON, US, Bd. 95, 1995, Seiten 1981-2001, XP002206526 ISSN: 0009-2665

## Beschreibung

Zur Behandlung von bakteriellen Infektionskrankheiten wird eine große Zahl von Antibiotika therapeutisch eingesetzt. Die Krankheitserreger werden aber zunehmend resistent gegen die verwendeten Arzneimittel, es droht sogar eine große Gefahr durch sogenannte multiresistente Keime, die nicht nur gegen einzelne Antibiotikagruppen, wie z. B. β-Lactam-Antibiotika, Glycopeptide oder Macrolide widerstandsfähig geworden sind, sonderen gleichzeitig mehrere Resistenzen tragen. Es gibt sogar Krankheitserreger, die gegen alle im Handel erhältlichen Antibiotika resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antibiotika beschrieben worden, die meisten sind jedoch zu toxisch, um als Arzneimittel eingesetzt werden zu können.

Es ist bereits eine größere Anzahl von Antibiotika mit Tetramsäure-Grundstruktur beschrieben worden. Die Tetramsäure, das 2,4-Pyrrolidindion, ist die Stammverbindung von verschiedenen Naturstoffen, die von einigen Mikroorganismen und marinen Invertebraten gebildet werden. Beschrieben wurde 1994 die
• Harziansäure (R. Sawa et al., J. Antibiotics, 47, 731-732, 1994), ein sehr wenig wirksames Antibiotikum;
   In einen zusammenfassenden Artikel von B.J. L. Royles werden die bis 1994 veröffentlichten natürlichen Tetramsäure-Derivate beschrieben (Chem. Rev. 95, Seite 1981 - 2001, 1995). Seit 1995 wurden weitere natürliche Tetramsäuren beschrieben, die zum Teil antibakterielle Eigenschaften aufweisen:
• Reutericyclin (A. Höltzel et al., Angew. Chem. 112, 2886-2888, 2000), eine schwach antibakteriell aktive Verbindung;
• Equisetin und Phomasetin (S. S. Singh et al., Tetrahedron Lett. 39, 2243-2246, 1998) sind isomere Inhibitoren der HIV-1 Integrase;
• Cryptocin (J. Y. Li et al., Org. Lett. 2, 767-770, 2000), eine antimycotische Verbindung;
• Vancoresmycin (N. V. S. Ramakrishna et al., Int. Patent Publikation Nr. WO 0028064), ein Antibiotikum;
• Coniosetin (L. Vertesy et al., Deutsche Patentanmeldung Nr. DE 10060810.8), ein wirksames Antibiotikum bestehend aus einem Tetramsäure und einem NaphthylTeil.

Es wurde überraschend gefunden worden, dass der Stamm ST 003236 (DSM 14476) das neue Antibiotikum Gabusectin zu bilden vermag, welches nicht nur antibakteriell sehr wirksam, sondern auch gut verträglich ist.

Gegenstand der Erfindung sind demzufolge die von dem Stamm ST 003236 (DSM 14476) gebildeten Verbindungen sowie deren physiologisch verträglichen Salze, Stereoisomere, Tautomere, Derivate, insbesondere Ester-Derivate, und offensichtliche chemische Äquivalente, wie beispielsweise Ether.

### Gegenstand der Erfindung sind Verbindungen der Formel (I)

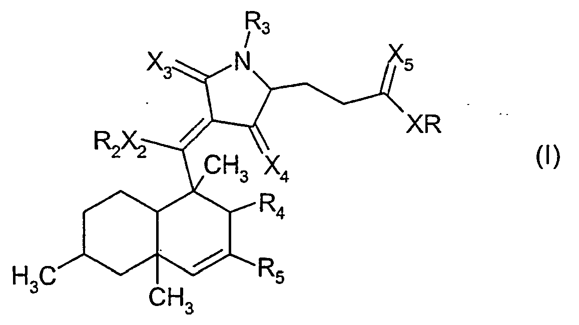

wobei
R, R₂ und R₃ unabhängig voneinander:
   1. H, oder
   2. C₁-C₆-Alkyl, C₂-C₆-Alkenyl oder C₂-C₆-Alkinyl bedeutet, worin Alkyl, Alkenyl und Alkinyl geradkettig oder verzweigt sind und gegebenenfalls ein- oder zweifach substituiert sind durch:
      - 2.1: -OH,
      - 2.2: =O,
      - 2.3: -O-C₁-C₆-Alkyl, worin Alkyl geradkettig oder verzweigt ist,
      - 2.4: -O-C₂-C₆-Alkenyl, worin Alkenyl geradkettig oder verzweigt ist,
      - 2.5: -Aryl,
      - 2.6: -NH-C₁-C₆-Alkyl, worin Alkyl geradkettig oder verzweigt ist,
      - 2.7: -NH-C₂-C₆-Alkenyl, worin Alkenyl geradkettig oder verzweigt ist,
      - 2.8: -NH₂ oder
      - 2.9: Halogen,
   worin die Substituenten 2.3 bis 2.7 weiter substituiert sein können durch -CN, - Amid oder -Oxim-Funktionen,
   - R₄: C₁-C₆-Alkyl oder C₂-C₆-Alkenyl bedeutet, worin Alkyl und Alkenyl geradkettig oder verzweigt sein kann und gegebenenfalls ein- oder zweifach substituiert ist, wie unter 2.1 bis 2.9 beschrieben,
   - R₅: H oder Methyl,
   X, X₂, X₃, X₄ und X₅, unabhängig voneinander O, NH, N-C₁-C₆-Alkyl, N-C₂-C₆-Alkenyl, N-C₂-C₆-Alkinyl, N-Acyl, N-Aryl, N-O-R oder S
   bedeuten,
   oder eine stereoisomere Form oder eine tautomere Form der Verbindung der Formel (I) oder ein Gemisch der vorgenannten Formen in jedem Verhältnis, oder ein physiologisch verträgliches Salz der Verbindung der Formel (I) oder einer stereoisomeren Form oder einer tautomeren Form einer Verbindung der Formel (I).

C₁-C₆-Alkyl bedeutet ein geradkettiges oder verzweigtes Alkyl mit 1 bis 6 C-Atomen, bevorzugt mit 1 bis 4 C-Atomen, z.B. Methyl, Ethyl, i-Propyl, tert.Butyl und Hexyl.

C₂-C₆-Alkenyl bedeutet ein geradkettiges oder verzweigtes Alkenyl mit 2 bis 6 C-Atomen, das einfach, zweifach oder dreifach ungesättigt ist, z.B. Allyl, Crotyl, 1-Propenyl, Penta-1,3-dienyl und Pentenyl.

C₂-C₆-Alkinyl bedeutet ein geradkettiges oder verzweigtes Alkinyl mit 2 bis 6 C-Atomen, das einfach oder zweifach ungesättigt ist, z.B. Propinyl, Butinyl und Pentinyl.

Aryl bedeutet Phenyl, Benzyl oder 1- oder 2- Naphthyl, daß auch noch substituiert sein kann, beispielsweise durch Halogen, wie Chlor, Brom, Fluor, durch Alkyl mit 1-4 C-Atomen, vorzugsweise Methyl, durch Hydroxy, durch Alkoxy mit 1-4 C Atomen, insbesondere Methoxy oder durch Trifluormethyl stehen.

Acyl kann für aliphatische oder aromatische Acyl-Reste stehen. Aliphatisches Acyl hat 1-7, vorzugsweise 1-4 C Atome, wie z.B. Formyl, Acetyl, Propionyl, Butyryl, Hexanoyl, Acryloyl, Crotonoyl, Propioloyl, das noch weiter substituiert sein kann, beispielsweise durch Halogen, wie Chlor, Brom, Fluor, durch Amino, oder durch Alkylamino mit 1-4 C Atomen, vorzugsweise Methyl- oder Ethylamino-Gruppen. Aromatisches Acyl kann z.B. Benzoyl oder Naphthoyl sein, das auch noch weiter substituiert sein kann, beispielsweise durch Halogen, wie Chlor, Brom, Fluor, durch Alkyl mit 1-4 C-Atomen, vorzugsweise Methyl, durch Hydroxy-, durch Amino-Gruppen, wie z.B. Ethylamino-, oder durch Alkoxy-Gruppen mit 1-7, vorzugsweise 1-4C Atomen, insbesondere Methoxy.

Vorzugsweise betrifft die Erfindung eine Verbindung der Formel (I), wobei
- R: 1.0 H, oder
2.0 C₁-C₆-Alkyl, C₂-C₆-Alkenyl oder C₂-C₆-Alkinyl, worin Alkyl, Alkenyl und Alkinyl geradkettig oder verzweigt sind und gegebenenfalls ein- oder zweifach substituiert sind durch: 2.1-OH, 2.2 =O, 2.3-O-C₁-C₆-Alkyl, worin Alkyl geradkettig oder verzweigt ist, 2.4-O-C₂-C₆-Alkenyl, worin Alkenyl geradkettig oder verzweigt ist, 2.5-Aryl, 2.6-NH-C₁-C₆-Alkyl, worin Alkyl geradkettig oder verzweigt ist, 2.7-NH-C₂-C₆-Alkenyl, worin Alkenyl geradkettig oder verzweigt ist, 2.8-NH₂ oder 2.9 Halogen, worin die Substituenten 2.3 bis 2.7 weiter substituiert sein können durch -CN, -Amid oder-Oxim-Funktionen,
- R₂: H,
- R₃: CH₃,
- R₄: -CH=CH-CH₃,
- R₅: CH₃, und
- X, X₂, X₃, X₄ und X₅: O bedeuten.

Besonders bevorzugt betrifft die Erfindung eine Verbindung der Formel (I), wobei
- R: H,
- R₂: H oder CH₃,
- R₃: CH3,
- R₄: -CH=CH-CH₃,
- R₅: CH₃, und
- X, X₂, X₃, X₄ und X₅: O bedeuten.

Tautomere Formen der Verbindung (I) sind beispielweise eine Verbindung der Formel (II) 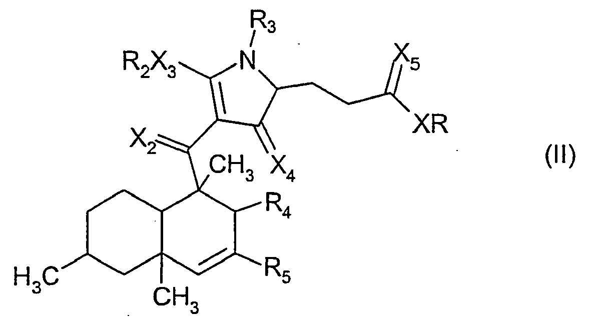 wobei die Reste R, R₂, R₃, R₄, R₅, X, X₂, X₃, X₄ und X₅ wie oben definiert sind, wobei tautomere Formen der Verbindungen der Formel (I) beispielsweise aus dem Wasserstoff-verbrückten Tetramsäure-Strukturteil resultieren, 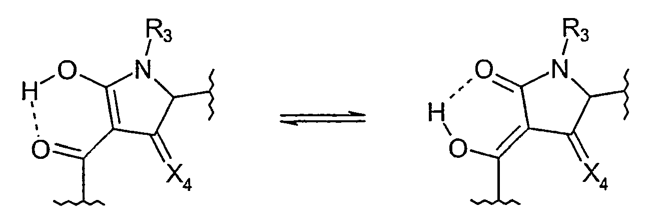 und sich in Lösung in Abhängigkeit von Parametern wie beispielsweise pH-Wert und Lösungsmittelpolarität ineinander um wandeln.

Chiralitätszentren in den Verbindungen der Formeln (I) und (II) können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Von den erfindungsgemäßen Verbindungen der Formeln (I) und (II) sind solche Verbindungen bevorzugt, in denen die Konfiguration dem substituierten hydrierten Naphthyl-Gerüst der Formel (III) entspricht:

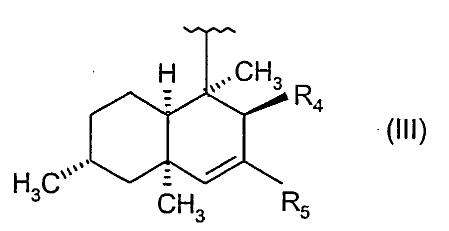

Die Erfindung betrifft des weiteren eine Verbindung der Formel (IV), 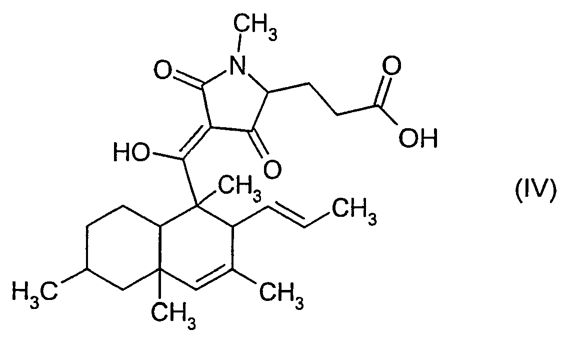 eine Verbindung der Formel (V), 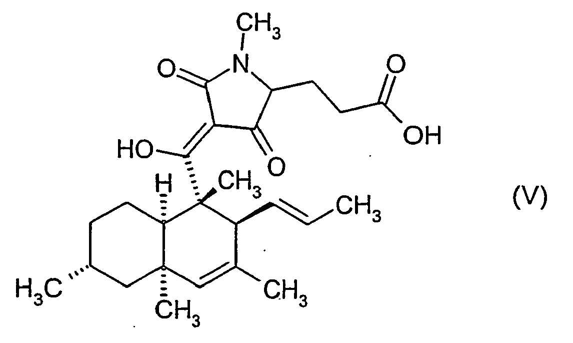 eine Verbindung der Formel (VI), 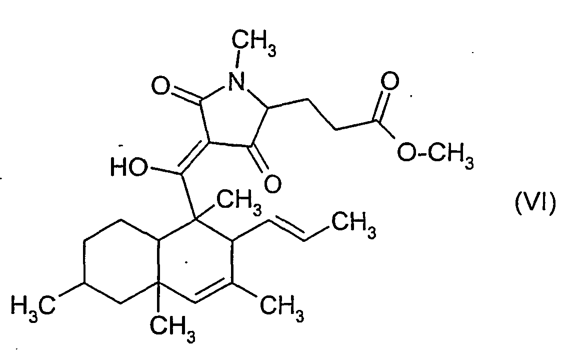 eine Verbindung der Formel (VII), 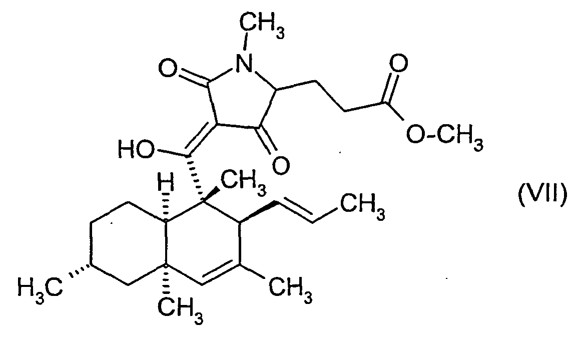 oder eine stereoisomere Form oder eine tautomere Form einer Verbindung der Formel (IV), (V), (VI) oder (VII) oder ein Gemisch der jeweiligen vorgenannten Formen in jedem Verhältnis, oder ein physiologisch verträgliches Salz einer Verbindung der Formel (IV), (V), (VI) oder (VII) oder einer stereoisomeren Form oder einer tautomeren. Form einer Verbindung der Formel (IV), (V), (VI) oder (VII).

Die erfinderischen Verbindungen unterscheiden sich von literaturbekannten Substanzen beispielsweise durch die Polarität, ihre chemische Struktur oder ihre antimikrobiellen Wirksamkeit oder weitere physikalischen Eigenschaften. Insbesondere enthalten die erfindungsgemäßen Verbindungen gegenüber den Verbindungen im Stand der Technik eine zusätzliche Methylgruppe im Naphthylteil.

Die Erfindung betrifft weiterhin offensichtliche chemische Äquivalente der Verbindungen der Formeln (I) bis (VII).
Offensichtliche chemische Äquivalente der erfindungsgemäßen Verbindungen sind Verbindungen, die die gleiche Wirksamkeit wie die erfindungsgemäßen Verbindungen haben und einen geringfügigen chemischen Unterschied aufweisen oder sich unter milden Bedingungen in die erfindungsgemäßen Verbindungen umwandeln. Zu den genannten Äquivalenten gehören z.B. Ester, Azomethine (Schiff'sche Basen), Ketale, Oxime, Hydrierungsprodukte, Reduktionsprodukte, Komplexe oder Addukte der bzw. mit den erfindungsgemäßen Verbindungen.

Beispielsweise kann eine aktivierte Säure, zum Beispiel Säurechloride oder andere Säure-Derivate, mit der Hydroxygruppe der Verbindung der Formel (I) umgesetzt werden, oder eine oder mehrere Doppelbindungen und/oder Carbonylgruppen der Verbindung der Formel (I) kann mit einem Reduktionsmittel reduziert werden, wobei Doppelbindungen zum Beispiel mit H₂/Pd und Carbonylgruppen zum Beispiel mit NaBH₄ reduziert werden. Die oben genannten Methoden zur Derivatisierung sind in Lehrbüchern wie Jerry March, Advanced Organic Chemistry, John Wiley & Sons, 4th Edition, 1992, beschrieben. Um Umsetzungen selektiv durchzuführen, kann es vorteilhaft sein, in an sich bekannter Weise vor der Reaktion geeignete Schutzgruppen einzuführen. Die Schutzgruppen werden nach der Reaktion abgespalten, anschließend wird das Reaktionsprodukt gereinigt.

Die Erfindung betrifft desweiteren Gabusectin, eine Verbindung der Summelformel C₂₅H₃₅NO₄, nachgewiesen durch ESI- und FAB-Massenspektroskopie, und charakterisiert durch die ¹H-NMR- und ¹³C-NMR-Daten gemäß Tabelle 2, oder eine stereoisomere Form oder eine tautomere Form der Verbindung Gabusectin oder ein Gemisch der jeweiligen vorgenannten Formen in jedem Verhältnis, oder ein physiologisch verträgliches Salz der Verbindung Gabusectin oder einer stereoisomeren Form oder einer tautomeren Form der Verbindung Gabusectin.

Die Erfindung betrifft desweiteren Gabusectin-Methylester, eine Verbindung der Summelformel C₂₇H₃₉NO₅, nachgewiesen ESI- und FAB-Massenspektroskopie, und charakterisiert durch die ¹H-NMR- und ¹³C-NMR-Daten gemäß Tabelle 3, oder eine stereoisomere Form oder eine tautomere Form der Gabusectin-Methylester oder ein Gemisch der jeweiligen vorgenannten Formen in jedem Verhältnis, oder ein physiologisch verträgliches Salz der Verbindung Gabusectin-Methylester oder einer stereoisomeren Form oder einer tautomeren Form der Verbindung Gabusectin-Methylester.

Die Erfindung betrifft des weiteren eine Verbindung Formel (I) erhältlich durch Fermentierung von ST 003236 (DSM 14476) oder einer Variante und/oder Mutanten von ST 003236 (DSM 14476) in einem Kulturmedium, bis sich die Verbindung der Formel (I) in der Kulturbrühe anhäuft, anschließender Isolierung der Verbindung der Formel (I), sowie gegebenenfalls Überführung in ein pharmakologisch verträgliches Salz.

Die Erfindung betrifft des weiteren eine Verbindung der Summenformel C₂₆H₃₇NO₅ (Gabusectin) erhältlich durch Fermentierung von ST 003236 (DSM 14476) oder eine Variante und/oder Mutante von ST 003236 (DSM 14476) in einem Kulturmedium, bis sich die Verbindung Gabusectin in der Kulturbrühe anhäuft, anschließender Isolierung der Verbindung Gabusectin, sowie gegebenenfalls Überführung in ein pharmakologisch verträgliches Salz.

Weiterhin betrifft die Erfindung ein Verfahren zur Herstellung der Verbindung der Formel (I), dadurch gekennzeichnet, daß der Mikroorganismus ST 003236 (DSM 14476) oder eine Variante und/oder Mutante von ST 003236 (DSM 14476) in einem wäßrigen Nährmedium kultiviert wird, eine Verbindung der Formel (I) isoliert und gereinigt wird und gegebenenfalls in ein offensichtliches chemisches Äquivalent oder ein pharmakologisch verträgliches Salz überführt wird.

Die Erfindung betrifft desweiteren ein Verfahren zur Herstellung einer Verbindung der Formel (I), dadurch gekennzeichnet, daß Gabusectin der Formel (IV) mit einem C₁-C₆-Alkyl-, C₂-C₆-Alkenyl- oder C₂-C₆-Alkinyl-Alkoholderivat oder mit einem C₁-C₆-Alkyl-, C₂-C₆-Alkenyl- oder C₂-C₆-Alkinyl-Alkylierungsmittel zu einer Verbindung der Formel (I) verestert wird, worin Alkyl, Alkenyl und Alkinyl geradkettig oder verzweigt sind und gegebenenfalls ein- oder zweifach durch die Reste 2.1 bis 2.9 gemäß Formel (I) in Anspruch 1 substituiert sein können, worin die Substituenten 2.3 bis 2.7 weiter substituiert sein können durch -CN, Amid oder -Oxim-Funktionen, und R₂ H, R₃ CH₃, R₄ -CH=CH-CH₃, R₅ CH₃, und X, X₂, X₃, X₄ und X₅ O bedeuten, vorzugsweise mit einem C₁-C₆-Alkyl-Alkylierungsmittel, besonders bevorzugt mit einem C₁-Alkylierungsmittel.

C₁-C₆-Alkyl-, C₂-C₆-Alkenyl- oder C₂-C₆-Alkinyl-Alkoholderivate sind geradkettig oder verzweigt sind und gegebenenfalls ein- oder zweifach durch die Reste 2.1 bis 2.9 vide supra substituiert, worin die Substituenten 2.3 bis 2.7 weiter substituiert sein können durch -CN, -Amid oder -Oxim-Funktionen, beispielsweise Methanol, Ethanol, n-Propanol, Isopropanol, n-Butanol, sec.-Butanol, tert.-Butanol und n-Hexanol, 2-Buten-1-ol (Crotylalhohol), 1-Propen-3-ol (Allylalkohol), 1,3-Pentadien-5-ol, 1,4-Pentadien-3-ol und 2-Penten-1-ol , 1-Penten-4-ol (Allylmethylcarbinol), 1-Penten-3-ol (Ethylvinylcarbinol), 2-Propin-1-ol (Propargylalkohol), 1-Butin-3-ol, 2-Butin-1-ol, 3-Butin-1-ol, 1-Pentin-3-ol, 2-Pentin-1-ol, 3-Pentin-1-ol, 4-Pentin-1-ol, vorzugsweise Methanol.

C₁-C₆-Alkyl-, C₂-C₆-Alkenyl- oder C₂-C₆-Alkinyl-Alkylierungsmittel sind geradkettig oder verzweigt sind und gegebenenfalls ein- oder zweifach durch die Reste 2.1 bis 2.9 vide supra substituiert, beispielsweise Diazomethan-Derivate als C₁-Alkylierungsmittel, wie zum Beispiel Trimethylsilyldiazomethan.

Methoden zur Veresterung sind beispielsweise beschrieben in Jerry March, Advanced Organic Chemistry, John Wiley & Sons, 4th Edition, 1992.

Der Stamm ST 003236 wurde bei der Deutschen Sammlung von Mikroorganismen und Zellkulturen GmbH (DSM), Mascheroder Weg 1 B, 38124 Braunschweig, Deutschland nach den Regeln des Budapester Vertrages unter der folgenden Nummer hinterlegt DSM 14476.

Das besagte Verfahren umfasst die Kultivierung von ST 003236 (DSM 14476), seiner Mutanten oder Varianten unter aeroben Bedingungen in einem eine oder mehrere Kohlenstoff- und Stickstoffquellen, anorganischen Salze und gegebenenfalls Spurenelemente enthaltenden Kulturmedium.

Der Fermentationsverlauf und die Bildung der erfindungsgemäßen Antibiotika kann entsprechend dem Fachmann bekannten Methoden, wie z. B. durch Austestung der biologischen Aktivität in Bioassays oder durch chromatographische Methoden wie Dünnschichtchromatographie (DC) oder Hochleistungsflüssigkeits-Chromatographie (HPLC) verfolgt werden.

Eine Mutante ist ein Mikroorganismus, in dem ein oder mehrere Gene des Genoms modifiziert wurden, wobei das Gen oder die Gene funktionell und vererbbar erhalten bleiben, die für die Fähigkeit des Organismus verantwortlich sind, die erfinderische Verbindung zu produzieren.

Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden, oder wie von Brock et al. in "Biology of Microorganisms", Prentice Hall, Seite 238-247 (1984) beschrieben.

Eine Variante ist ein Phenotyp des Mikroorganismus. Mikroorganismen haben die Fähigkeit, sich an ihre Umgebung anzupassen und zeigen daher ausgeprägte physiologische Flexibilität. Bei der phenotypischen Anpassung sind alle Zellen des Mikroorganismus involviert, wobei die Art der Veränderung nicht genetisch konditioniert ist und unter veränderten Bedingungen reversibel ist (H. Stolp, Microbial ecology: organismus, habitats, activities. Cambridge University Press, Cambridge, GB, Seite 180, 1988).

Das Screening nach Mutanten und Varianten, die das erfindungsgemäße Antibiotikum produzieren, kann durch Bestimmung der biologischen Aktivität des in der Kulturbrühe angehäuften Wirkstoffes, beispielsweise durch Bestimmung der antibakteriellen Wirkung erfolgen, oder durch Detektion von Verbindungen, die als antibakteriell aktiv bekannt sind, in der Fermentationsbrühe durch beispielsweise HPLC-oder LC-MS-Methoden.

Die Verbindung Gabusectin kommt sowohl im Mycel als auch im Kulturfiltrat vor. Es ist deshalb zweckmäßig, die Fermentationslösung in das Kulturfiltrat und das Mycel durch Filtration zu trennen und getrennt zu trocknen. Das getrocknete Kulturfiltrat und das getrocknete Mycel werden zweckmäßiger Weise getrennt mit einem organischen Lösungsmittel, zum Beispiel Methanol oder Propanol-2 extrahiert.

Die Extraktion kann in einem weiten pH-Bereich durchgeführt werden, es ist jedoch zweckmäßig, im neutralen oder schwach sauren Milieu, vorzugsweise zwischen pH 3 und pH 7 zu arbeiten. Der Extrakt kann z. B. im Vakuum konzentriert und getrocknet werden.

Eine Methode der Isolierung des erfindungsgemäße Antibiotikums verläuft nach dem Prinzip Trennprinzip der Polarität in an sich bekannter Weise.

Eine weitere Methode der Reinigung ist die Chromatographie an Adsorptionsharzen wie z. B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Geeignet sind darüber hinaus zahlreiche Reverse-, Phase Träger, z. B. RP₈ und RP₁₈, wie sie z. B. im Rahmen der Hochdruckflüssigkeits-Chromatographie (HPLC) allgemein bekannt geworden sind.

Eine weitere Reinigungsmöglichkeit für die erfindungsgemäße Verbindung besteht in der Verwendung von sogenannten Normal-Phasen-Chromatographie-Trägern, wie z.B. Kieselgel oder Al₂O₃ oder anderen in an sich bekannter Weise.

Ein alternatives Isolierungsverfahren ist die Verwendung von Molekularsieben, wie z. B. Fractogel® TSK HW-40 (Merck, Deutschland) und andere, in an sich bekannter Weise. Es ist darüber hinaus auch möglich, aus angereichertem Material das Gabusectin durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 4 bis 10 und Kationenaustauschern vorzugsweise im pH-Bereich von 2 bis 5. Besonders geeignet ist hierfür die Verwendung von Pufferlösungen, denen man Anteile von organischen Lösungsmitteln hinzugefügt hat.
Gabusectin, die genannten chemischen Derivate davon sowie die offensichtlichen chemischen Äquivalente derselben können nach dem Fachmann bekannten Methoden in die entsprechenden pharmakologisch verträglichen Salze übergeführt werden

Unter pharmakologisch verträglichen Salzen der erfindungsgemäßen Verbindungen versteht man sowohl anorganische als auch organische Salze, wie sie in Remingtons Pharmaceutical Sciences (17. Auflage, Seite 1418 [1985]) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, H₂SO₄, Maleinsäure, Fumarsäure in Frage.

Es wurde überraschend gefunden, daß die erfindungsgemäßen Verbindungen der Formel (I) antibakterielle Wirkungen aufweisen und sich daher zur Therapie von Erkrankungen eignen, die durch bakterielle Infektionen verursacht werden. Tabelle 1 faßt die Minimalen Hemmkonzentrationen (MHK) von Gabusectin beispielhaft zusammen.

**Tabelle 1: Antibakterielle In-Vitro Aktivität der Verbindung Gabusectin im Reihenverdünnungstest.**

| | |
|---|---|
| Bakterium (Stamm) | MHK-Werte (µg/ml) |
| S.aureus (SG511) | 5 |
| S.aureus (Exp54146) | 20 |
| Bakterium (Stamm) | MHK-Werte (µg/ml) |
| S.pyogenes(A561) | 20 |
| E.faecium (M78L) | 40 |

Die Verträglichkeit des Gabusectin ist in der wirksamen Konzentration und darüber gut.

Die vorliegende Erfindung betrifft daher auch die Verwendung einer oder mehrerer der erfindungsgemäßen Verbindungen der Formeln (I) bis (VII) als Arzneimittel, sowie die Verwendung einer oder mehrerer der erfindungsgemäßen Verbindungen der Formeln (I) bis (VII) zur Herstellung von Arzneimitteln, insbesondere zur Behandlung und/oder zur Prophylaxe von bakteriellen Infektionen.

Des weiteren betrifft die vorliegende Erfindung ein Arzneimittel mit einem Gehalt an einer oder mehreren erfindungsgemäßen Verbindungen.

Das besagte Arzneimittel enthaltend eine Verbindung der Formel (I) wird mit einem oder mehreren physiologischen Hilfsstoffen hergestellt und in eine geeignete Darreichungsform gebracht.

Die erfindungsgemäßen Arzneimittel können enteral (oral), parenteral (intramuskulär oder intravenös), rektal oder lokal (topisch) angewendet werden. Sie können in Form von Lösungen, Pulvern (Tabletten, Kapseln einschließlich Mikrokapseln), Salben (Cremes oder Gel), oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Die folgenden Beispiele sollen der näheren Erläuterung der Erfindung dienen, ohne die Breite der Erfindung in irgendeiner Weise zu begrenzen.

### Beispiel 1 Herstellung einer Glycerinkultur von ST 003236 (DSM 14476).

30 mL Nährlösung (Malzextrakt 2.0%, Hefeextrakt 0.2 %, Glucose 1.0 % (NH₄)₂ HPO₄ 0.05 %, pH 6.0) in einem sterilen 100 mL Erlenmeyerkolben wurden mit dem Stamm ST 003236 (DSM 14476) beimpft und 6 Tage bei 25° C und 140 UpM auf einer rotierenden Schüttelmaschine inkubiert. 1.5 mL dieser Kultur wurden anschließend mit 2.5 mL 80 %igem Glycerin verdünnt und bei -135°C gelagert.

### Beispiel 2 Herstellung einer Vorkultur im Erlenmeyerkolben von ST 003236 (DSM 14476).

100 mL Nährlösung (Malzextrakt 2.0%, Hefeextract 0.2 %, Glucose 1.0 % (NH₄)₂ HPO₄ 0.05 %, pH 6.0) in einem sterilen 300 mL Erlenmeyerkolben wurden mit einer Ampulle des Stammes ST 003236 (DSM 14476) beimpft und 6 Tage bei 25° C und 140 UpM inkubiert. 2 mL dieser Vorkultur wurden anschließend für die Herstellung der Hauptkulturen beimpft.

### Beispiel 3 Herstellung einer flüssigen Hauptkultur ST 003236 (DSM 14476).

Ein steriler 300 mL Erlenmeyerkolben mit 100 mL der folgende Nährlösung Potato dextrose 2.4%, Hefeextrakt 0.2 %, wurde mit einer auf einem Schrägröhrchen (gleiche Nährlösung, jedoch mit 2 % Agar) gewachsenen Kultur oder mit 2 mL einer Vorkultur (s. Beispiel 2) beimpft und auf einer Schüttelmaschine bei 140 UpM und 25 C inkubiert. Die maximale Produktion einer oder mehrerer Verbindungen der erfindungsgemäßen Verbindungen der Formel (I) war nach ca. 144 Stunden erreicht. Zum Animpfen von 10 bis 200 L Fermentern genügte eine 96 Stunden alte Submerskultur (Animpfmenge ca. 10 %) aus der gleichen Nährlösung. Die Bedingungen für diese Fermenter waren:
Temperatur 25° C
Rührergeschwindigkeit: 200 UpM
Belüftung 15 L/min.
Durch wiederholte Zugabe von ethanolischer Polyollösung konnte die Schaumbildung unterdrückt werden. Das Produktionsrriaximum wurde nach ca. 96 bis 144 Stunden erreicht.

### Beispiel 4: Isolierung der Verbindung Gabusectin.

3 L Kulturlösung, gewonnen nach Beispiel 3, wurden filtriert und das Kulturfiltrat und das Mycel getrennt gefriergetrocknet. Das getrocknete Kulturfiltrat wurde mit 3 Liter Methanol extrahiert. Die klare flüssige Phase wurde im Vakuum auf 200 mL eingeengt und filtriert. Diese Methanollösung wurde mit Wasser in einer HPLC Hochdruckgradientenanlage 9 : 1 gemischt und auf eine 300 mL fassende, mit dem Adsorptionsharz MCI Gel® CHP20P (Mitsubishi Casei Corp., Tokyo) gefüllte Säule aufgetragen. Säulenmaße: Breite x Höhe: 5 cm x 15 cm. Eluiert wurde mit einem Lösungsmittel-Gradienten von Wasser bis 100 % Methanol und der Säulenausfluß (50 mL/Minute) in Fraktionen je 25 mL aufgefangen. Die Gabusectin-haltigen Fraktionen 65 bis 75, die durch HPLC-Analysen überprüft wurden, wurden gesammelt und im Vakuum konzentriert sowie gefriergetrocknet (0.23 g).

### Beispiel 5: Reinigung von Gabusectin durch Hochdruckflüssigkeitschromatographie (HPLC).

| | |
|---|---|
| Säule: | Purospher ® STAR RP-18 e 3 µm, 30-2, (Merck, Deutschland) |
| | |
| Mobile Phase Puffer A: | 5 % Acetonitril + 0.1 % Ammoniumacetat, |
| Mobile Phase Puffer B: | 95 % Acetonitril +0.1% Ammoniumacetat, |
| Gradient: | 15 min |
| Flußgeschwindigkeit: | 0.25 mL pro Minute |

Detektion durch UV-Absorption bei 210 nm.

Es wurde für Gabusectin die Retentionszeit von 6.5 Min. gefunden.

### Beispiel 6: Endreinigung von Gabusectin.

Das angereicherte Antibiotikum Gabusectin (0.23 g), gewonnen nach Beispiel 4, wurde auf einer LUNA® 10 µm C 18(2)-HPLC-Säule (Phenomenex, USA) (Breite x Höhe = 2.1 cm x 25 cm) im Gradientenverfahren mit 5 % bis 95 % Acetonitril in 0.05 % Trifluoressigsäure aufgetrennt. Fluß: 25 mL/Min. Fraktionsgröße: 25 mL. Die durch analytische HPLC (siehe Beispiel 5) untersuchte Fraktion 48 wurde gefriergetrocknet. Sie ergab 50 mg Gabusectin in 98 %iger Reinheit.

### Beispiel 7: Charakterisierung von Gabusectin.

Die physikalisch-chemischen sowie spektroskopischen Eigenschaften des erfindungsgemäßen Antibiotikums lassen sich wie folgt zusammenfassen: Aussehen:
Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.

| | |
|---|---|
| Summenformel: | C₂₆H₃₇NO₅ |
| Molekulargewicht: | 443,59 |
| ¹H- und ¹³C-NMR: | siehe Tabelle 2 |
| UV-Maxima: | 236 nm, 294 nm |

Bestimmung des Molpeaks:
Dem gesuchten Molekül wird die Masse 443 zugeordnet aufgrund folgender Befunde: ESI⁺-Spektrum sowie FAB⁺-Spektren zeigen Peaks bei 444 amu (M+H)⁺. ESI⁻-Spektrum zeigt einen Peak bei 442 amu (M-H)⁻. Hochauflösung des Quasi-Molekülions: FAB⁺ 444,27424 (M+H)⁺. Für die Summenformel C₂₆H₃₇NO₅ wurde 443,59 berrechnet.

**Tabelle 2: ¹H- und ¹³C- chemische Verschiebungen von Gabusectin in CDCl₃ bei 275K.**

| | | | |
|---|---|---|---|
| 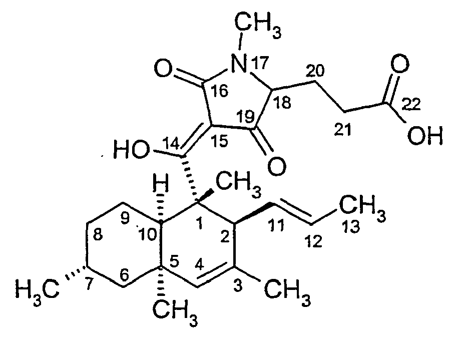 | | | |

| Position | ¹³C δ (ppm) | ¹H δ (ppm) | HMBC-Korrelationen (¹³C- ¹H) |
|---|---|---|---|
| 1 | 49.01 | - | H3-Me(w), H1-Me, H2(w), H10, 14-OH |
| 1-Me | 20.77 | 1.24 s | - |
| 2 | 45.69 | 3.36 | H3-Me, H9',H1-Me, H10, H4, H12(s), H 11 (w) |
| 3 | 132.86 | - | H11(w), H2, H3-Me(s), H6'(w) |
| 3-Me | 23.43 | 1.69 s | H4 |
| 4 | 130.04 | 5.06 | H2, H10, H3-Me(s), H6', H5-Me(s) |
| 5 | 37.61 | - | H6', H7-Me(w), H5-Me(s), H10, H4(s) |
| 5-Me | 31.91 | 0.70 | H3-Me, H6', H10(w) |
| 6 | 51.62 | 1.36,0.92 | H3-Me(w), H9', H7-Me, H5-Me, H4(w) |
| 7 | 29.46 | 1.26 | H6(w), H6', H9(w), H7-Me(s), H5-Me(w) |
| 7-Me | 22.41 | 0.81 d | - |
| 8 | 34.97 | 1.65,0.87 | H9(w), H9'(w), H6(w), H6', H7-Me(s) |
| 9 | 25.52 | 1.84, 1.29 | H10 |
| 10 | 42.17 | 2.66 dd | H9', H1-Me, H5-Me, H2(w), H4 |
| 11 | 132.25 | 5.31 | H12, H2(w), H13(s) |
| 12 | 128.95 | 5.44 | H11, H2, H13(s) |
| 13 | 17.90 | 1.69 | H12, H11 |
| 14 | 203.37 | - | 14-OH, H2, H10, H1-Me |
| 14-OH | - | 17.73 | - |
| 15 | 98.81 | - | 14-OH |
| 16 | 177.05 | - | 14-OH, H18(s), H17-Me(s) |
| 17-Me | 27.20 | 3.02 s | - |
| 18 | 64.27 | 3.76 dd | H17-Me, H20, H20' |
| 19 | 190.36 | - | H18, H20(w), H20' |
| 20 | 23.27 | 2.32,2.08 | H21, H18 |
| 21 | 27.44 | 2.30,2.30 | H20, H20', H18 |
| 22 | 178.18 | - | H20, H21 |
| 22-COOH | - | 7.1 br | - |

### Beispiel 8: Hemmwirkung des Gabusectin im Agar-Diffusionstest.

Es wurden Agarplatten zubereitet mit 2 mL Staphylococcus aureus Einsaat in 200 mL Agarlösung. Gabusectin wurde in einer 10 mM Lösung auf Blättchen mit einem Durchmesser von 6 mm aufgetragen und auf die Agarplatte gelegt. Die beimpften Staphylococcus-Platten wurden 16 Stunden bei 37°C inkubiert. Dann wurden Hemmhöfe mit folgenden Durchmessern (mm) beobachtet:

| Menge | Hemmhofgröße (mm) |
|---|---|
| 10 µL | 8 |
| 20 µL | 14 |
| 40 µL | 17 |

### Beispiel 9: Methylierung und anschließende Reinigung des Gabusectin-Methylesters.

20 mg Antibiotikum Gabusectin (0.045 mmol), gewonnen nach Beispiel 6, wurden in 5 mL MeOH gelöst und mit Trimethylsilyldiazomethan im 6-fachen molaren Überschuß versetzt. Die Reaktionsmischung wurde 60 min bei Raumtemperatur stehen gelassen und anschließend zur Trockne eingeengt. Das erhaltene Gemisch wurde chromatograhpisch mit einer LUNA® 5 µm C 18(2)-HPLC-Säule (Phenomenex, USA) (Breite x Höhe = 1cm x 25 cm) im Gradientenverfahren mit 5 % bis 95 % Acetonitril in 0.05 % Trifluoressigsäure aufgetrennt. Fluß: 6,5 mL/Min. Fraktionsgröße: 6,5 mL. Die durch analytische HPLC (siehe Beispiel 5) untersuchte Fraktion 61 wurde gefriergetrocknet. Sie ergab 7,4 mg Gabusectin-Methylester in 97 %iger Reinheit.

### Beispiel 10: Charakterisierung von Gabusectin-Methylester.

Die physikalisch-chemischen sowie spektroskopischen Eigenschaften des erfindungsgemäßen Antibiotikums lassen sich wie folgt zusammenfassen:
Aussehen:
Farblose bis hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.

| | |
|---|---|
| Summenformel: | C₂₇H₃₉NO₅ |
| Molekulargewicht: | 457,62 |
| ¹H- und ¹³C-NMR: | siehe Tabelle 3 |
| UV-Maxima: | 236 nm, 294 nm |

Bestimmung des Molpeaks:
Dem gesuchten Molekül wird die Masse 457,6 zugeordnet aufgrund folgender Befunde: ESI⁺-Spektrum sowie FAB⁺-Spektren zeigen Peaks bei 457 amu (M+H)⁺. ESI -Spektrum zeigt einen Peak bei 458,5 amu (M-H)-.

**Tabelle 3: ¹H- und ¹³C- chemische Verschiebungen von Gabusectin-Methylester in CDCl₃ bei 275K**

| | | | |
|---|---|---|---|
| 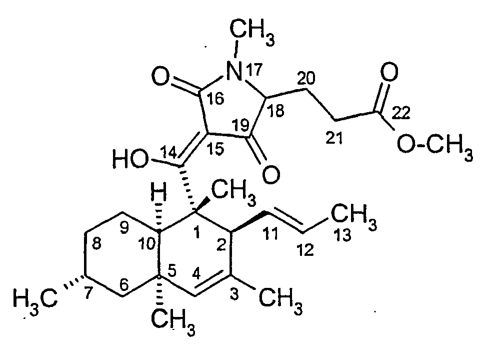 | | | |

| Position | ¹³C δ (ppm) | ¹H δ (ppm) | HMBC-Korrelationen (¹³C- ¹H) |
|---|---|---|---|
| 1 | 48.89 | - | H3-Me(w), H1-Me, H10(w) |
| 1-Me | 20.83 | 1.24 | - |
| 2 | 45.70 | 3.36 | H3-Me, H1-Me, H9', H10(w), H12, H4 |
| 3 | 132.86 | - | H3-Me |
| 3-Me | 23.44 | 1.68 | H4 |
| 4 | 130.08 | 5.05 | H3-Me, H6', H5-Me, H10(w) |
| 5 | 37.63 | - | H6', H5-Me, H10(w), H4(w) |
| 5-Me | 31.91 | 0.70 | H10(w), H6', H3-Me(w) |
| 6 | 51.65 | 1-35,0.92 | H9', H7-Me, H5-Me, H4(w) |
| 7 | 29.48 | 1.25 | H6', H7-Me |
| 7-Me | 22.42 | 0.80 | - |
| 8 | 35.00 | 1.64,0.88 | H6', H7-Me(s) |
| 9 | 25.55 | 1.83, 1.30 | H10 |
| 10 | 42.17 | 2.67 | H1-Me, H5-Me, H9', H4(w) |
| 11 | 132.35 | 5.30 | H12, H13 |
| 12 | 128.87 | 5.43 | H11, H13 |
| 13 | 17.90 | 1.69 | H12, H11 |
| 14 | 202.86 | - | H1-Me |
| 14-OH | - | 17.75 | - |
| 15 | 98.91 | - | - |
| 16 | 177.06 | - | H17-Me |
| 17-Me | 27.13 | 3.02 | - |
| 18 | 64.49 | 3.71 | H20, H20', H21, H17-Me |
| 19 | 190.33 | - | H18, H20' |
| 20 | 23.52 | 2.29, 2.19 | H21 |
| 21 | 27.42 | 2.23, 2.23 | - |
| 22 | 173.05 | - | 22-OMe, H20, H20', H21 |
| 22-OMe | 51.93 | 3.66 | - |

### Beispiel 11: Hemmwirkung des Gabusectin-Methylester im Agar-Diffusionstest.

Es wurden Agarplatten zubereitet mit 2 mL Staphylococcus aureus -Einsaat in 200 mL Agarlösung. Gabusectin-Methylester wird in einer 10 mM Lösung auf Blättchen mit einem Durchmesser von 6 mm aufgetragen und auf die Agarplatte gelegt. Die beimpften Staphylococcus-Platten wurden 16 Stunden bei 37°C inkubiert. Dann wurden Hemmhöfe mit folgenden Durchmessern (mm) beobachtet.

| Menge | Hemmhofgröße (mm) |
|---|---|
| 10 µL | 0 |
| 20 µL | 7 |
| 40 µL | 8 |

## Patentansprüche

1. Verbindung der Formel (I) 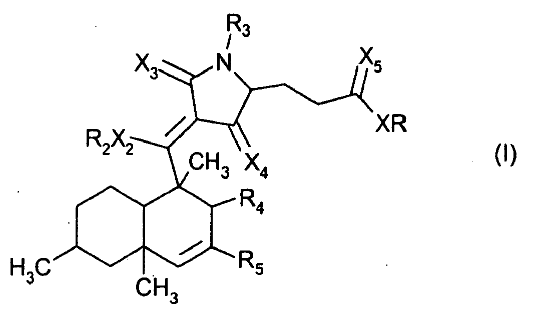 wobei
R, R₂ und R₃ unabhängig voneinander:
1. H, oder
2. C₁-C₆-Alkyl, C₂-C₆-Alkenyl oder C₂-C₆-Alkinyl bedeuten, worin Alkyl, Alkenyl und Alkinyl geradkettig oder verzweigt sind und gegebenenfalls ein- oder zweifach substituiert sind durch:
2.1 -OH,
2.2 =O,
2.3 -O-C₁-C₆-Alkyl, worin Alkyl geradkettig oder verzweigt ist,
2.4 -O-C₂-C₆-Alkenyl, worin Alkenyl geradkettig oder verzweigt ist,
2.5 -Aryl,
2.6 -NH-C₁-C₆-Alkyl, worin Alkyl geradkettig oder verzweigt ist,
2.7 -NH-C₂-C₆-Alkenyl, worin Alkenyl geradkettig oder verzweigt ist,
2.8 -NH₂ oder
2.9 Halogen,
worin die Substituenten 2.3 bis 2.7 weiter substituiert sein können durch -CN, - Amid oder -Oxim-Funktionen,
R₄ C₁-C₆-Alkyl oder C₂-C₆-Alkenyl bedeutet, worin Alkyl und Alkenyl geradkettig oder verzweigt sein kann und gegebenenfalls ein- oder zweifach substituiert ist, wie unter 2.1 bis 2.9 beschrieben,
R₅ H oder Methyl,
X, X₂, X₃, X₄ und X₅, unabhängig voneinander O, NH, N-C₁-C₆-Alkyl, N-C₂-C₆-Alkenyl, N-C₂-C₆-Alkinyl, N-Acyl, N-Aryl, N-O-R oder S
bedeuten,
oder eine stereoisomere Form oder eine tautomere Form der Verbindung der Formel (I) oder ein Gemisch der vorgenannten Formen in jedem Verhältnis, oder ein physiologisch verträgliches Salz der Verbindung der Formel (I) oder einer stereoisomeren Form oder einer tautomeren Form einer Verbindung der Formel (I).

2. Verbindung der Formel (I) gemäß Anspruch 1, wobei
R 1.0 H, oder
2.0 C₁-C₆-Alkyl, C₂-C₆-Alkenyl oder C₂-C₆-Alkinyl, worin Alkyl, Alkenyl und Alkinyl geradkettig oder verzweigt sind und gegebenenfalls ein- oder zweifach substituiert sind durch:
2.1 -OH,
2.2 =O,
2.3 -O-C₁-C₆-Alkyl, worin Alkyl geradkettig oder verzweigt ist,
2.4 -O-C₂-C₆-Alkenyl, worin Alkenyl geradkettig oder verzweigt ist,
2.5 -Aryl,
2.6 -NH-C₁-C₆-Alkyl, worin Alkyl geradkettig oder verzweigt ist,
2.7 -NH-C₂-C₆-Alkenyl, worin Alkenyl geradkettig oder verzweigt ist,
2.8 -NH₂ oder
2.9 Halogen,
worin die Substituenten 2.3 bis 2.7 weiter substituiert sein können durch -CN, -Amid oder-Oxim-Funktionen,
R₂ H,
R₃ CH₃,
R₄ -CH=CH-CH₃,
R₅ CH₃, und
X, X₂, X_{3,} X₄ und X₅ O bedeuten.

3. Verbindung der Formel (I) gemäß Anspruch 1, wobei
R H,
R₂ H oder CH₃,
R₃ CH₃,
R₄ -CH=CH-CH₃,
R₅ CH, und
X, X₂, X₃. X₄ und X₅ O bedeuten.

4. Verbindung der Formel (IV) gemäß Anspruch 1, 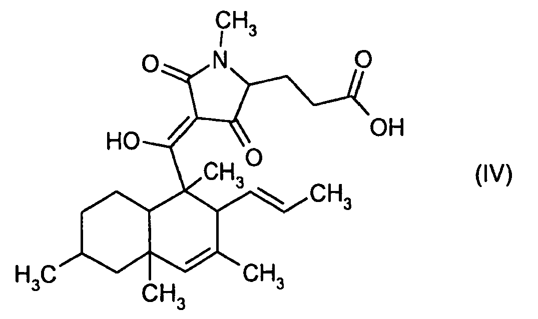 oder eine stereoisomere Form oder eine tautomere Form einer Verbindung der Formel (IV) oder ein Gemisch der jeweiligen vorgenannten Formen in jedem Verhältnis, oder ein physiologisch verträgliches Salz einer Verbindung der Formel (IV) oder einer stereoisomeren Form oder einer tautomeren Form einer Verbindung der Formel (IV).

5. Verbindung der Formel (V) gemäß Anspruch 1, 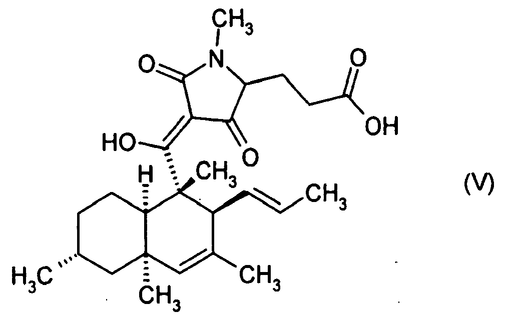 oder eine stereoisomere Form oder eine tautomere Form einer Verbindung der Formel (V) oder ein Gemisch der jeweiligen vorgenannten Formen in jedem Verhältnis, oder ein physiologisch verträgliches Salz einer Verbindung der Formel (V) oder einer stereoisomeren Form oder einer tautomeren Form einer Verbindung der Formel (V).

6. Verbindung der Formel (VI) gemäß Anspruch 1, 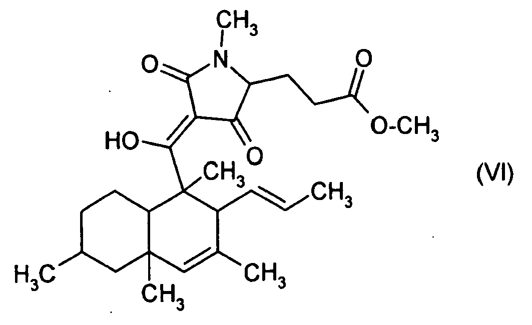 oder eine stereoisomere Form oder eine tautomere Form einer Verbindung der Formel (VI) oder ein Gemisch der jeweiligen vorgenannten Formen in jedem Verhältnis, oder ein physiologisch verträgliches Salz einer Verbindung der Formel (VI) oder einer stereoisomeren Form oder einer tautomeren Form einer Verbindung der Formel (VI).

7. Verbindung der Formel (VII) gemäß Anspruch 1, 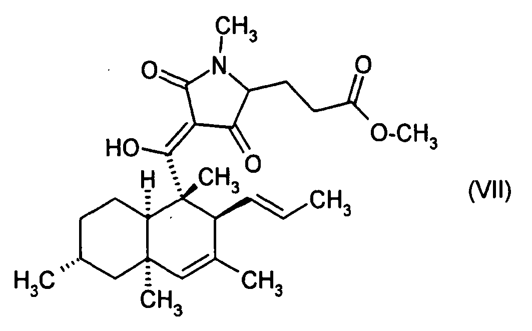 oder eine stereoisomere Form oder eine tautomere Form einer Verbindung der Formel (VII) oder ein Gemisch der jeweiligen vorgenannten Formen in jedem Verhältnis, oder ein physiologisch verträgliches Salz einer Verbindung der Formel (VII) oder einer stereoisomeren Form oder einer tautomeren Form einer Verbindung der Formel (VII).

8. Verbindung der Formel (I) gemäß Anspruch 1, **gekennzeichnet durch** die Summelformel C₂₅H₃₅NO₄, nachgewiesen **durch** ESI- und FAB-Massenspektroskopie, und charakterisiert **durch** die ¹H-NMR-Daten δ (CDCl₃, 275K) = 0.70, 0.81, 0.87, 0.92, 1.24, 1.26, 1.29, 1.36, 1.65, 1.69, 1.84, 2.08, 2.30, 2.32, 2.66, 3.02, 3.36, 3.76, 5.06, 5.31, 5.44, 7.1, 17.73, und die ¹³C-NMR-Daten δ (CDCl₃, 275K) = 17.90, 20.77, 22.41, 23.27, 23.43, 25.52, 27.20, 27.44, 29.46, 31.91, 34.97, 37.61, 42.17, 45.69, 49.01, 51.62, 64.27, 98.81, 128.95, 130.04, 132.25, 132.86, 177.05, 178.18, 190.36, 203.37, oder eine stereoisomere Form oder eine tautomere Form oder ein Gemisch der jeweiligen vorgenannten Formen in jedem Verhältnis, oder ein physiologisch verträgliches Salz oder einer stereoisomeren Form oder einer tautomeren Form davon.

9. Verbindung der Formel (I) gemäß Anspruch 1, **gekennzeichnet durch** die Summelformel C₂₇H₃₉NO₅, nachgewiesen ESI- und FAB-Massenspektroskopie, und charakterisiert **durch** die ¹H-NMR-Daten δ (CDCl₃, 275K) = 0.70, 0.80, 0.88, 0.92, 1.24, 1.25, 1.30, 1.35, 1.64, 1.68, 1.69, 1.83, 2.11, 2.23, 2.29, 2.67, 3.02, 3.36, 3.66, 3.71, 5.05, 5.30, 5.43, 17.75 und die ¹³C-NMR-Daten δ (CDCl₃, 275K) = 17.90, 20.83, 22.42, 23.44, 23.52, 25.55, 27.13, 27.42, 29.48, 31.91, 35.00, 37.63, 42.17, 45.70, 48.89, 51.65, 51.93, 64.49, 98.91, 128.87, 130.08, 132.35, 132.86, 173.05, 177.06, 190.33, 202.86, oder eine stereoisomere Form oder eine tautomere Form oder ein Gemisch der jeweiligen vorgenannten Formen in jedem Verhältnis, oder ein physiologisch verträgliches Salz oder einer stereoisomeren Form oder einer tautomeren Form davon.

10. Verbindung Formel (I) gemäß Anspruch 1, erhältlich durch Fermentierung von ST 003236 (DSM 14476) oder eine Variante und/oder Mutante von ST 003236 (DSM 14476) in einem Kulturmedium, bis sich die Verbindung der Formel (I) in der Kulturbrühe anhäuft, anschließender Isolierung der Verbindung der Formel (I), sowie gegebenenfalls Überführung in ein pharmakologisch verträgliches Salz.

11. Verbindung der Formel (I) gemäß Anspruch 1, **gekennzeichnet durch** die Summenformel C₂₆H₃₇NO₅ erhältlich **durch** Fermentierung von ST 003236 (DSM 14476) oder eine Variante und/oder Mutante von ST 003236 (DSM 14476) in einem Kulturmedium, bis sich die Verbindung in der Kulturbrühe anhäuft, anschließender Isolierung, sowie gegebenenfalls Überführung in ein pharmakologisch verträgliches Salz.

12. Verfahren zur Herstellung der Verbindung der Formel (I) gemäß Anspruch 1, **dadurch gekennzeichnet, daß** der Mikroorganismus ST 003236 (DSM 14476) oder eine Variante und/oder Mutante von ST 003236 in einem wäßrigen Nährmedium kultiviert wird, eine Verbindung der Formel (I) isoliert und gereinigt wird und gegebenenfalls in ein offensichtliches chemisches Äquivalent und/oder ein pharmakologisch verträgliches Salz überführt wird.

13. Verfahren gemäß Anspruch 12, **dadurch gekennzeichnet, daß** der Mikroorganismus ST 003236 (DSM 14476) oder eine Mutante und/oder Variante von ST 003236 in einem eine Kohlenstoff- und Stickstoffquelle sowie die üblichen anorganischen Salze und Spurenelemente enthaltenden Kulturmedien unter aeroben Bedingungen fermentiert.

14. Verfahren gemäß einem oder mehreren der Ansprüche 12 und 13, **dadurch gekennzeichnet, daß** die Fermentation unter aeroben Bedingungen bei einer Temperatur zwischen 20 und 35°C und bei einem pH zwischen 4 und 10 durchgeführt wird.

15. Verfahren zur Herstellung einer Verbindung der Formel (I) gemäß Anspruch 1, **dadurch gekennzeichnet, daß** Gabusectin der Formel (IV) gemäß Anspruch 4 mit einem C₁-C₆-Alkyl-, C₂-C₆-Alkenyl- oder C₂-C₆-Alkinyl-Alkoholderivat oder mit einem C₁-C₆-Alkyl-, C₂-C₆-Alkenyl- oder C₂-C₆-Alkinyl-Alkylierungsmittel zu einer Verbindung der Formel (I) gemäß Anspruch 1 verestert wird, worin Alkyl, Alkenyl und Alkinyl geradkettig oder verzweigt sind und gegebenenfalls ein- oder zweifach durch die Reste 2.1 bis 2.9 substituiert sein können, worin die Substituenten 2.3 bis 2.7 weiter substituiert sein können durch -CN, Amid oder -Oxim-Funktionen, und R₂ H, R₃ CH₃, R₄ -CH=CH-CH₃, R₅ CH₃, und X, X₂, X₃, X₄ und X₅ O bedeuten.

16. Verfahren zur Herstellung einer Verbindung der Formel (I) gemäß Anspruch 15, wobei mit einem C₁-Alkylierungsmittel verestert wird.

17. Verwendung einer Verbindung gemäß einem oder mehreren der Ansprüche 1 bis 11 zur Herstellung eines Arzneimittels.

18. Verwendung einer Verbindung gemäß einem oder mehreren der Ansprüche 1 bis 11 zur Herstellung eines Arzneimittels zur Behandlung und Prophylaxe von bakteriellen Infektionskrankheiten.

19. Arzneimittel mit einem Gehalt an mindestens einer Verbindung gemäß einem oder mehreren der Ansprüche 1 bis 11 und einem oder mehreren physiologisch geeigneten Hilfsstoffen.

20. Verfahren zur Herstellung eines Arzneimittels gemäß Anspruch 19, **dadurch gekennzeichnet, daß** man mindestens eine Verbindung gemäß einem oder mehreren der Ansprüche 1 bis 11 mit einem oder mehreren physiologisch geeigneten Hilfsstoffen in eine geeignete Darreichungsform bringt.

21. Mikroorganismus ST003236 (DSM 14476).

## Claims

1. A compound of the formula (I) 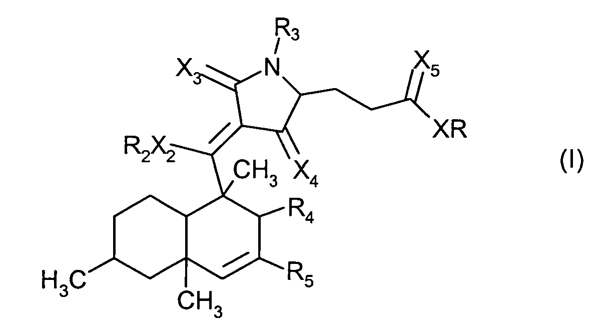 where
R, R₂ and R₃ are, independently of each other:
1. H, or
2. C₁-C₆-alkyl, C₂-C₆-alkenyl or C₂-C₆-alkynyl, in which alkyl, alkenyl and alkynyl are straight-chain or branched and are optionally substituted, once or twice, by:
2.1 -OH,
2.2 =O,
2.3 -O-C₁-C₆-alkyl, in which alkyl is straight- chain or branched,
2.4 -O-C₂-C₆-alkenyl, in which alkenyl is straight-chain or branched,
2.5 -aryl,
2.6 -NH-C₁-C₆-alkyl, in which alkyl is straight- chain or branched,
2.7 -NH-C₂-C₆-alkenyl, in which alkenyl is straight-chain or branched,
2.8 -NH₂ or
2.9 halogen,
in which the substituents 2.3 to 2.7 can be further substituted by -CN, -amide or -oxime functions,
R₄ is C₁-C₆-alkyl or C₂-C₆-alkenyl, in which alkyl and alkenyl can be straight-chain or branched and are optionally substituted once or twice, as described under 2.1 to 2.9,
R₅ is H or methyl,
X, X₂, X₃, X₄ and X₅, are independently of each other, O, NH, N-C₁-C₆-alkyl, N-C₂-C₆-alkenyl, N-C₂-C₆-alkynyl, N-acyl, N-aryl, N-O-R or S,
or a stereoisomeric form or a tautomeric form of the compound of the formula (I) or a mixture of the previously mentioned forms in any ratio, or a physiologically tolerated salt of the compound of the formula (I) or of a stereoisomeric form or of a tautomeric form of a compound of the formula (I).

2. A compound of the formula (I) as claimed in claim 1, where
R is 1.0 H, or
2.0 C₁-C₆-alkyl, C₂-C₆-alkenyl or C₂-C₆-alkynyl, in which alkyl, alkenyl and alkynyl are straight-chain or branched and are optionally substituted once or twice by:
2.1 -OH,
2.2 =O,
2.3 -O-C₁-C₆-alkyl, in which alkyl is straight-chain or branched,
2.4 -O-C₂-C₆-alkenyl, in which alkenyl is straight-chain or branched,
2.5 -aryl,
2.6 -NH-C₁-C₆-alkyl, in which alkyl is straight-chain or branched,
2.7 -NH-C₂-C₆-alkenyl, in which alkenyl is straight-chain or branched,
2.8 -NH₂ or
2.9 halogen,
in which the substituents 2.3 to 2.7 can be further substituted by -CN, -amide or - oxime functions,
R₂ is H,
R₃ is CH₃,
R₄ is -CH=CH-CH₃,
R₅ is CH₃, and
X, X₂, X₃, X₄ and X₅ are O.

3. A compound of the formula (I) as claimed in claim 1, where
R is H,
R₂ is H or CH₃,
R₃ is CH₃,
R₄ is -CH=CH-CH₃,
R₅ is CH₃, and
X, X₂, X₃, X₄ and X₅ are O.

4. A compound of the formula (IV) as claimed in claim 1, 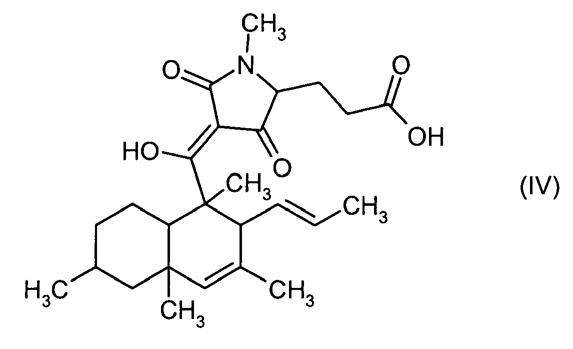 or a stereoisomeric form or a tautomeric form of a compound of the formula (IV) or a mixture of the respective previously mentioned forms in any ratio, or a physiologically tolerated salt of a compound of the formula (IV) or of a stereoisomeric form or of a tautomeric form of a compound of the formula (IV).

5. A compound of the formula (V) as claimed in claim 1, 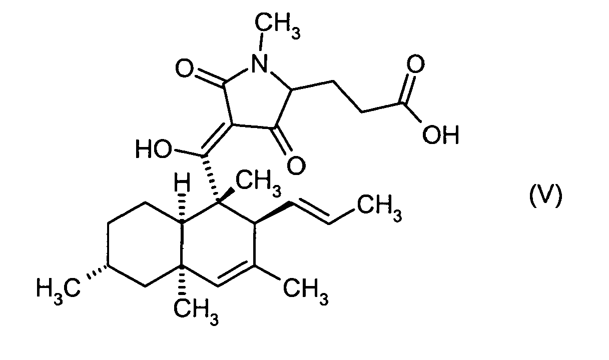 or a stereoisomeric form or a tautomeric form of a compound of the formula (V) or a mixture of the respective previously mentioned forms in any ratio, or a physiologically tolerated salt of a compound of the formula (V) or of a stereoisomeric form or of a tautomeric form of a compound of the formula (V).

6. A compound of the formula (VI) as claimed in claim 1, 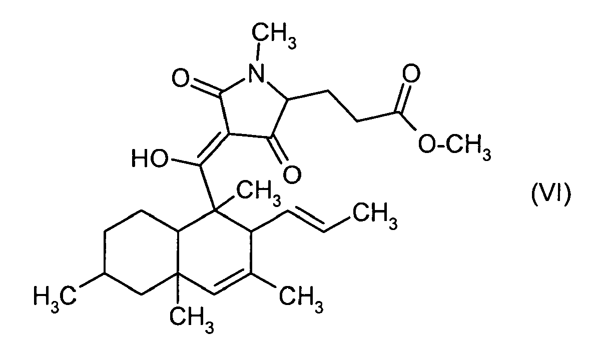 or a stereoisomeric form or a tautomeric form of a compound of the formula (VI) or a mixture of the respective previously mentioned forms in any ratio, or a physiologically tolerated salt of a compound of the formula (VI) or of a stereoisomeric form or of a tautomeric form of a compound of the formula (VI).

7. A compound of the formula (VII), as claimed in claim 1, 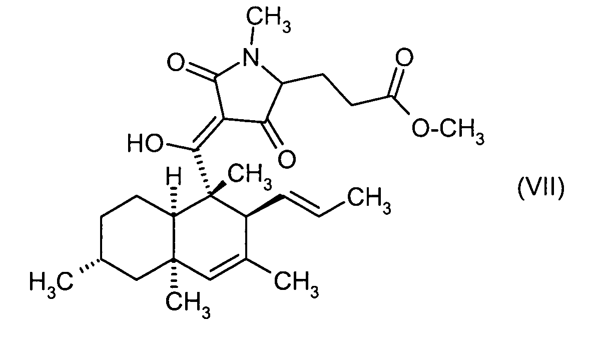 or a stereoisomeric form or a tautomeric form of a compound of the formula (VII) or a mixture of the respective previously mentioned forms in any ratio, or a physiologically tolerated salt of a compound of the formula (VII) or of a stereoisomeric form or of a tautomeric form of a compound of the formula (VII).

8. A compound of the formula (I) as claimed in claim 1, **characterized by** the empirical formula C₂₅H₃₅NO₄, demonstrated by ESI and FAB mass spectroscopy, and **characterized by** the ¹H NMR data δ (CDCl₃, 275K) = 0.70, 0.81, 0.87, 0.92, 1.24, 1.26, 1.29, 1.36, 1.65, 1.69, 1.84, 2.08, 2.30, 2.32, 2.66, 3.02, 3.36, 3.76, 5.06, 5.31, 5.44, 7.1, 17.73, and the ¹³C NMR data δ (CDCl₃, 275K) = 17.90, 20.77, 22.41, 23.27, 23.43, 25.52, 27.20, 27.44, 29.46, 31.91, 34.97, 37.61, 42.17, 45.69, 49.01, 51.62, 64.27, 98.81, 128.95, 130.04, 132.25, 132.86, 177.05, 178.18, 190.36, 203.37, or a stereoisomeric form or a tautomeric form or a mixture of the respective previously mentioned forms in any ratio, or a physiologically tolerated salt or of a stereoisomeric form or of a tautomeric form thereof.

9. A compound of the formula (I) as claimed in claim 1, **characterized by** the empirical formula C₂₇H₃₉NO₅, demonstrated by ESI and FAB mass spectroscopy, and **characterized by** the ¹H NMR data δ (CDCl₃, 275K) = 0.70, 0.80, 0.88, 0.92, 1.24, 1.25, 1.30, 1.35, 1.64, 1.68, 1.69, 1.83, 2.11, 2.23, 2.29, 2.67, 3.02, 3.36, 3.66, 3.71, 5.05, 5.30, 5.43, 17.75 and the ¹³C NMR data δ (CDCl₃, 275K) = 17.90, 20.83, 22.42, 23.44, 23.52, 25.55, 27.13, 27.42, 29.48, 31.91, 35.00, 37.63, 42.17, 45.70, 48.89, 51.65, 51.93, 64.49, 98.91, 128.87, 130.08, 132.35, 132.86, 173.05, 177.06, 190.33, 202.86, or a stereoisomeric form or a tautomeric form or a mixture of the respective previously mentioned forms in any ratio, or a physiologically tolerated salt or of a stereoisomeric form or of a tautomeric form thereof.

10. A compound of the formula (I) as claimed in claim 1, obtainable by fermenting ST 003236 (DSM 14476), or a variant and/or mutant of ST 003236 (DSM 14476) in a culture medium until the compound of the formula (I) accumulates in the culture broth, subsequently isolating the compound of the formula (I) and, where appropriate, converting it into a pharmacologically tolerated salt.

11. A compound of the formula (I) as claimed in claim 1, **characterized by** the empirical formula C₂₆H₃₇NO₅ obtainable by fermenting ST 003236 (DSM 14476) or a variant and/or mutant of ST 003236 (DSM 14476), in a culture medium until the compound accumulates in the culture broth, subsequently isolating said compound and, where appropriate, converting it into a pharmacologically tolerated salt.

12. A process for preparing the compound of the formula (I) as claimed in claim 1, which comprises culturing the microorganism ST 003236 (DSM 14476), or a variant and/or mutant of ST 003236 in an aqueous nutrient medium, isolating and purifying a compound of the formula (I), and, where appropriate, converting it into an obvious chemical equivalent and/or a pharmacologically tolerated salt.

13. The process as claimed in claim 12, wherein the microorganism ST 003236 (DSM 14476) or a mutant and/or variant of ST 003236 is fermented, under aerobic conditions, in a culture medium which contains a carbon source and a nitrogen source and also the customary inorganic salts and trace elements.

14. The process as claimed in either or both claims 12 and 13, wherein the fermentation under aerobic conditions is carried out at a temperature between 20 and 35°C and at a pH of between 4 and 10.

15. A process for preparing a compound of the formula (I) as claimed in claim 1, which comprises esterifying gabusectin of the formula (IV) as claimed in claim 4, with a C₁-C₆-alkyl-, C₂-C₆-alkenyl- or C₂-C₆-alkynyl-alcohol derivative or with a C₁-C₆-alkyl-, C₂-C₆-alkenyl- or C₂-C₆-alkynyl-alkylating agent to give a compound of the formula (I) as claimed in claim 1, in which alkyl, alkenyl and alkynyl are straight-chain or branched and can be optionally substituted once or twice by the radicals 2.1 to 2.9, in which the substituents 2.3 to 2.7 can be further substituted by - CN, -amide or -oxime functions, and R₂ is H, R₃ is CH₃, R₄ is -CH=CH-CH₃, R₅ is CH₃, and X, X₂, X₃, X₄ and X₅ are O.

16. The process for preparing a compound of the formula (I) as claimed in claim 15, wherein the esterification is carried out using a C₁-alkylating agent.

17. The use of a compound as claimed in one or more of claims 1 to 11 for producing a pharmaceutical.

18. The use of a compound as claimed in one or more of claims 1 to 11 for producing a pharmaceutical for the treatment and prophylaxis of infectious diseases caused by bacteria.

19. A pharmaceutical having a content of at least one compound as claimed in one or more of claims 1 to 11 and of one or more physiologically suitable auxiliary substances.

20. A process for producing a pharmaceutical as claimed in claim 19, which comprises bringing at least one compound as claimed in one or more of claims 1 to 11, together with one or more physiologically suitable auxiliary substances, into a suitable administration form.

21. The microorganism ST003236 (DSM 14476).

## Revendications

1. Composé de formule (I) 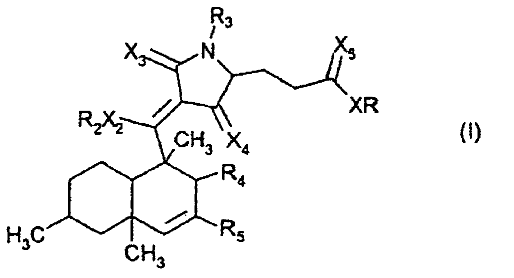 où
R, R₂ et R₃, indépendamment l'un de l'autre, signifient :
1. H, ou
2. C₁-C₆-alkyle, C₂-C₆-alcényle ou C₂-C₆-alcynyle, où alkyle, alcényle et alcynyle sont linéaires ou ramifiés et sont le cas échéant monosubstitués ou disubstitués par :
2.1 -OH,
2.2 =O,
2.3 -O-C₁-C₆-alkyle, où alkyle est linéaire ou ramifié,
2.4 -O-C₂-C₆-alcényle, où alcényle est linéaire ou ramifié,
2.5 -aryle,
2.6 -NH-C₁-C₆-alkyle, où alkyle est linéaire ou ramifié,
2.7 -NH-C₂-C₆-alcényle, où alcényle est linéaire ou ramifié,
2.8 -NH₂ ou
2.9 halogène,
où les substituants 2.3 à 2.7 peuvent en outre être substitués par des fonctions -CN, -amide ou -oxime,
R₄ signifie C₁-C₆-alkyle ou C₂-C₆-alcényle, où alkyle et alcényle peuvent être linéaires ou ramifiés et sont le cas échéant monosubstitués ou disubstitués comme décrit aux points 2.1 à 2.9,
R₅ signifie H ou méthyle,
X, X₂, X₃, X₄ et X₅, signifient indépendamment l'un de l'autre O, NH, N-C₁-C₆-alkyle, N-C₂-C₆-alcényle, N-C₂-C₆-alcynyle, N-acyle, N-aryle, N-O-R ou S,
ou une forme stéréo-isomère ou une forme tautomère du composé de formule (I) ou un mélange des formes susmentionnées dans n'importe quel rapport ou un sel physiologiquement tolérable du composé de formule (I) ou d'une forme stéréo-isomère ou d'une forme tautomère d'un composé de formule (I).

2. Composé de formule (I) selon la revendication 1, avec les significations suivantes
R 1.0 H, ou
2.0 C₁-C₆-alkyle, C₂-C₆-alcényle ou C₂-C₆-alcynyle, où alkyle, alcényle et alcynyle sont linéaires ou ramifiés et sont le cas échéant monosubstitués ou disubstitués par :
2.1 -OH,
2.2 =O
2.3 -O-C₁-C₆-alkyle, où alkyle est linéaire ou ramifié,
2.4 -O-C₂-C₆-alcényle, où alcényle est linéaire ou ramifié,
2.5 -aryle,
2.6 -NH-C₁-C₆-alkyle, où alkyle est linéaire ou ramifié,
2.7 -NH-C₂-C₆-alcényle, où alcényle est linéaire ou ramifié,
2.8 -NH₂ ou
2.9 halogène,
où les substituants 2.3 à 2.7 peuvent en outre être substitués par des fonctions -CN, -amide ou -oxime,
R₂ H,
R₃ CH₃,
R₄ -CH=CH-CH₃,
R₅ CH₃, et
X, X₂, X₃, X₄ et X₅ O.

3. Composé de formule (I) selon la revendication 1, avec les significations suivantes
R H,
R₂ H ou CH₃,
R₃ CH₃,
R₄ -CH=CH-CH₃,
R₅ CH₃, et
X, X₂, X₃, X₄ et X₅ O.

4. Composé de formule (IV) selon la revendication 1, 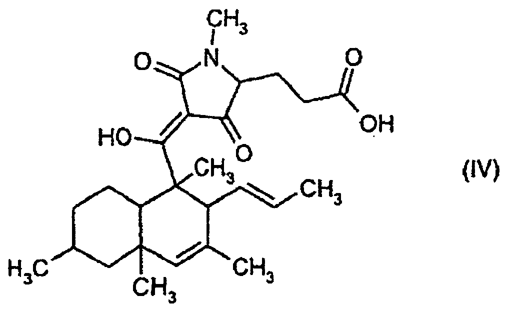 ou une forme stéréo-isomère ou une forme tautomère d'un composé de formule (IV) ou un mélange des différentes formes susmentionnées dans n'importe quel rapport ou un sel physiologiquement tolérable d'un composé de formule (IV) ou d'une forme stéréo-isomère ou d'une forme tautomère d'un composé de formule (IV).

5. Composé de formule (V) selon la revendication 1, 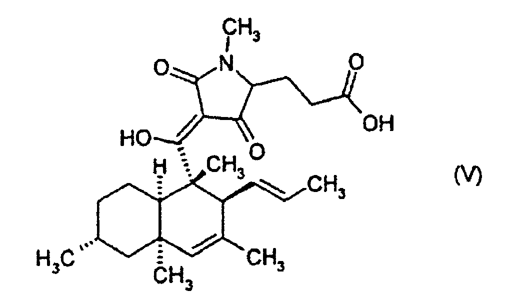 ou une forme stéréo-isomère ou une forme tautomère d'un composé de formule (V) ou un mélange des différentes formes susmentionnées dans n'importe quel rapport ou un sel physiologiquement tolérable d'un composé de formule (V) ou d'une forme stéréo-isomère ou d'une forme tautomère d'un composé de formule (V).

6. Composé de formule (VI) selon la revendication 1, 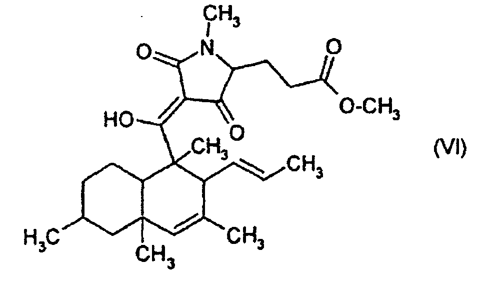 ou une forme stéréo-isomère ou une forme tautomère d'un composé de formule (VI) ou un mélange des différentes formes susmentionnées dans n'importe quel rapport ou un sel physiologiquement tolérable d'un composé de formule (VI) ou d'une forme stéréo-isomère ou d'une forme tautomère d'un composé de formule (VI).

7. Composé de formule (VII) selon la revendication 1, 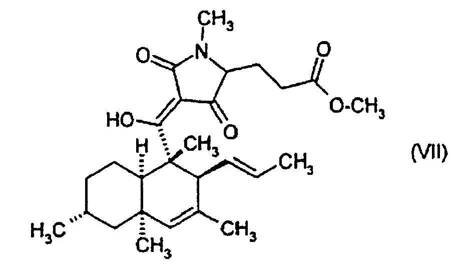 ou une forme stéréo-isomère ou une forme tautomère d'un composé de formule (VII) ou un mélange des différentes formes susmentionnées dans n'importe quel rapport ou un sel physiologiquement tolérable d'un composé de formule (VII) ou d'une forme stéréo-isomère ou d'une forme tautomère d'un composé de formule (VII).

8. Composé de formule (I) selon la revendication 1, **caractérisé par** la formule brute C₂₅H₃₅NO₄, prouvée par spectroscopie de masse ESI et FAB et **caractérisé par** les données ¹H-RMN δ (CDCl₃, 275K) = 0,70, 0,81, 0,87, 0,92, 1,24, 1,26, 1,29, 1,36, 1,65, 1,69, 1,84, 2,08, 2,30, 2,32, 2,66, 3,02, 3,36, 3,76, 5,06, 5,31, 5,44, 7,1, 17,73, et les données ¹³C-RMN δ (CDCl₃, 275K) = 17,90, 20,77, 22,41, 23,27, 23,43, 25,52, 27,20, 27,44, 29,46, 31,91, 34,97, 37,61, 42,17, 45,69, 49,01, 51,62, 64,27, 98,81, 128,95, 130,04, 132,25, 132,86, 177,05, 178,18, 190,36, 203,37, ou une forme stéréo-isomère ou une forme tautomère ou un mélange des différentes formes susmentionnées dans n'importe quel rapport ou un sel physiologiquement tolérable ou une forme stéréo-isomère ou une forme tautomère de celui-ci.

9. Composé de formule (I) selon la revendication 1, **caractérisé par** la formule brute C₂₇H₃₉NO₅, prouvée par spectroscopie de masse ESI et FAB et **caractérisé par** les données ¹H-RMN δ (CDCl₃, 275K) = 0,70, 0,80, 0,88, 0,92, 1,24, 1,25, 1,30, 1,35, 1,64, 1,68, 1,69, 1,83, 2,11, 2,23, 2,29, 2,67, 3,02, 3,36, 3,66, 3,71, 5,05, 5,30, 5,43, 17,75 et les données ¹³C-RMN δ (CDCl₃, 275K) = 17,90, 20,83, 22,42, 23,44, 23,52, 25,55, 27,13, 27,42, 29,48, 31,91, 35,00, 37,63, 42,17, 45,70, 48,89, 51,65, 51,93, 64,49, 98,91, 128,87, 130,08, 132,35, 132,86, 173,05, 177,06, 190,33, 202,37, ou une forme stéréo-isomère ou une forme tautomère ou un mélange des différentes formes susmentionnées dans n'importe quel rapport ou un sel physiologiquement tolérable ou une forme stéréo-isomère ou une forme tautomère de celui-ci.

10. Composé de formule (I) selon la revendication 1, pouvant être obtenu par fermentation de ST 003236 (DSM 14476) ou d'un variant et/ou d'un mutant de ST 003236 (DSM 14476) dans un bouillon de culture, jusqu'à ce que le composé de formule (I) s'accumule dans le bouillon de culture, isolement consécutif du composé de formule (I), ainsi que le cas échéant conversion en un sel pharmacologiquement tolérable.

11. Composé de formule (I) selon la revendication 1, **caractérisé par** la formule brute C₂₆H₃₇NO₅, pouvant être obtenu par fermentation de ST 003236 (DSM 14476) ou d'un variant et/ou d'un mutant de ST 003236 (DSM 14476) dans un bouillon de culture, jusqu'à ce que le composé de formule (I) s'accumule dans le bouillon de culture, isolement consécutif, ainsi que le cas échéant conversion en un sel pharmacologiquement tolérable.

12. Procédé pour la préparation du composé de formule (I) selon la revendication 1, **caractérisé en ce que** le microorganisme ST 003236 (DSM 14476) ou un variant et/ou un mutant de ST 003236 est cultivé dans un milieu nutritif aqueux, un composé de formule (I) est isolé et purifié et le cas échéant transféré en un équivalent chimique manifeste et/ou un sel pharmacologiquement tolérable.

13. Procédé selon la revendication 12, **caractérisé en ce que** le microorganisme ST 003236 (DSM 14476) ou un mutant et/ou un variant de ST 003236 est fermenté dans un bouillon de culture contenant une source de carbone et d'azote ainsi que les sels inorganiques et les oligoéléments usuels dans des conditions aérobies.

14. Procédé selon l'une ou plusieurs des revendications 12 à 13, **caractérisé en ce que** la fermentation est réalisée dans des conditions aérobies à une température entre 20 et 35°C et à un pH entre 4 et 10.

15. Procédé pour la préparation d'un composé de formule (I) selon la revendication 1, **caractérisé en ce que** de la gabusectine de formule (IV) selon la revendication 4 est estérifiée avec un dérivé d'un alcool C₁-C₆-alkylique, C₂-C₆-alcénylique ou C₂-C₆-alcynylique ou avec un agent d'alkylation C₁-C₆-alkylique, C₂-C₆-alcénylique ou C₂-C₆-alcynylique en un composé de formule (I) selon la revendication 1, où alkyle, alcényle et alcynyle sont linéaires ou ramifiés et peuvent être le cas échéant monosubstitués ou disubstitués par les radicaux 2.1 à 2.9, les substituants 2.3 à 2.7 pouvant être substitués en outre par des fonctions -CN, -amide ou -oxime, et R₂ signifie H, R₃ signifie CH₃, R₄ signifie -CH=CH-CH₃, R₅ signifie CH₃, et X, X₂, X₃, X₄ et X₅ signifient O.

16. Procédé pour la préparation d'un composé de formule (I) selon la revendication 15, où on estérifie avec un agent d'alkylation en C₁.

17. Utilisation d'un composé selon l'une ou plusieurs des revendications 1 à 11 pour la préparation d'un médicament.

18. Utilisation d'un composé selon l'une ou plusieurs des revendications 1 à 11 pour la préparation d'un médicament destiné au traitement et/ou à la prophylaxie de maladies infectieuses bactériennes.

19. Médicament présentant une teneur en au moins un composé selon l'une ou plusieurs des revendications 1 à 11 et un ou plusieurs adjuvants physiologiquement appropriés.

20. Procédé pour la préparation d'un médicament selon la revendication 19, **caractérisé en ce qu'**on amène au moins un composé selon l'une ou plusieurs des revendications 1 à 11 avec un ou plusieurs adjuvants physiologiquement appropriés dans une forme d'administration appropriée.

21. Microorganisme ST003236 (DSM 14476).
